# EUROPEAN PATENT SPECIFICATION

(11) **EP 3 047 814 B1**
(45) Date of publication and mention of the grant of the patent: **24.07.2019**
(21) Application number: 16158108.7
(22) Date of filing: 28.12.2011
(51) Int. Cl.: A61B 18/14, A61B 17/29

(54) **APPARATUS FOR PERFORMING AN ELECTROSURGICAL PROCEDURE**
VORRICHTUNG ZUR DURCHFÜHRUNG EINES ELEKTROCHIRURGISCHEN VERFAHRENS
APPAREIL POUR EXÉCUTER UNE PROCÉDURE ÉLECTROCHIRURGICALE

(30) Priority: 30.12.2010 US 981787
(43) Date of publication of application: 27.07.2016
(62) Divisional of application: 11195872.4
(73) Proprietor: Covidien LP, New Haven, CT 06511 (US)
(72) Inventor: Twomey, John R., Longmont, CO 80501 (US); Allen, James D., IV, Broomfield, CO 80020 (US)
(74) Representative: Morgan, Marc

(56) References cited:
- US-A- 5 496 347
- US-A- 5 509 922
- US-A- 5 919 206
- US-A1- 2007 260 242
- US-A1- 2010 076 433

## Description

### BACKGROUND

### Technical Field

The present disclosure relates to an apparatus for performing an electrosurgical procedure. More particularly, the present disclosure relates to an electrosurgical apparatus including an end effector assembly having a pair of jaw members that provide a mechanical advantage at the end effector.

### Description of Related Art

Electrosurgical instruments, e.g., electrosurgical forceps (open or closed type), are well known in the medical arts and typically include a housing, a handle assembly, a shaft and an end effector assembly attached to a distal end of the shaft. The end effector includes jaw members configured to manipulate tissue (e.g., grasp and seal tissue). Typically, the electrosurgical forceps utilizes both mechanical clamping action and electrical energy to effect hemostasis by heating the tissue and blood vessels to coagulate, cauterize, seal, cut, desiccate, and/or fulgurate tissue. Typically, one or more driving mechanisms, e.g., a drive assembly including a drive element, is utilized to cooperate with one or more components operatively associated with the end effector to impart movement to one or both of the jaw members.

To insulate the shaft and/or operative components, e.g., wiring, contained therein, a heat shrink may encase the shaft. Typically, the heat shrink extends to a distal end of the shaft adjacent the end effector.

To facilitate moving the jaw members from an open position for grasping tissue to a closed position for clamping tissue (or vice versa) such that a consistent, uniform tissue effect (e.g., tissue seal) is achieved, one or more types of suitable devices may be operably associated with the electrosurgical forceps. For example, in some instances, one or more cam members, e.g., a cam pin, may operably couple to the drive element, e.g., a drive rod, wire, cable, etc., and operably couple to a cam slot that is operably associated with one or both of the jaw members. Typically, the cam slots are operably disposed at proximal ends of the jaw members. In certain instances, to facilitate movement of the jaw members, the proximal ends of the jaw members are configured to extend outside of the shaft profile. In this extended position, the proximal ends of the jaw members are commonly referred to as "flags." Because the "flags" are configured to extend past the shaft profile, the heat shrink, typically, does extend past the proximal ends of the jaw members. Consequently, when the "flags" are in the extended position, the wiring that is coupled to the jaw members may be exposed to the surgical environment.

As can be appreciated, exposing the wiring to the surgical environment may result in damage to the wire, which, in turn, may decrease the operative life of the forceps.

In addition to the foregoing, the shaft may bend or deform during the course of an electrosurgical procedure. For example, under certain circumstances, a clinician may intentionally bend or articulate the shaft to gain a desired mechanical advantage at the surgical site. Or, under certain circumstances, the surgical environment may cause unintentional or unwanted bending or flexing of the shaft, such as, for example, in the instance where the shaft is a component of a catheter-based electrosurgical forceps. More particularly, shafts associated with catheter-based electrosurgical forceps are typically designed to function with relatively small jaw members, e.g., jaw members that are configured to pass through openings that are 3mm or less in diameter. Accordingly, the shaft and operative components associated therewith, e.g., a drive rod, are proportioned appropriately. That is, the shaft and drive rod are relatively small.

As can be appreciated, when the shaft is bent or deformed (either intentionally or unintentionally) the frictional losses associated with "flags" extending through the shaft profile may be transferred to one of the drive rod, drive element, and/or a spring operably associated with the drive assembly, which, in turn, may diminish, impede and/or prevent effective transfer of the desired closure force that is needed at the jaw members. Moreover, the frictional losses may also lessen the operative life of the spring, which, in turn, may ultimately lessen the operative life of the electrosurgical instrument. US5919206 discloses a surgical tool for grasping and cutting pieces of food lodged in oesophagus.

### SUMMARY

The present disclosure provides an endoscopic forceps. The endoscopic forceps includes a housing having a shaft that extends therefrom and defines a longitudinal axis therethrough. The shaft includes a stationary cam pin at a distal end thereof and an elongated cam slot operably disposed adjacent and in proximal relation relative to the stationary cam pin. An end effector assembly operatively connects to a distal end of the shaft and includes a pair of first and second jaw members pivotably coupled to one another. One of the first and second jaw members movable relative to the other jaw member from an open position, wherein the first and second jaw members are disposed in spaced relation relative to one another, to a clamping position, wherein the first and second jaw members cooperate to grasp tissue therebetween. The movable jaw member has a drive pin operably coupled thereto that is movable within the cam slot on the shaft from a proximal position that corresponds to the movable jaw member being in the clamping position, to a distal position that corresponds to the movable jaw member being in the open position. The movable jaw member has a second cam slot operably disposed thereon and operably coupled to the stationary cam pin on the shaft.

The present disclosure provides endoscopic forceps. The endoscopic forceps includes a housing having a shaft that extends therefrom and defines a longitudinal axis therethrough. The shaft includes a stationary cam pin at a distal end thereof and an elongated cam slot operably disposed adjacent and in proximal relation relative to the stationary cam pin. An end effector assembly operatively connects to a distal end of the shaft and includes a pair of first and second jaw members pivotably coupled to one another. One of the first and second jaw members is both translatable along the longitudinal axis and rotatable about the stationary cam pin, from an open position, wherein the first and second jaw members are disposed in spaced relation relative to one another, to a clamping position, wherein the first and second jaw members cooperate to grasp tissue therebetween. The movable jaw member has a drive pin operably defined therethrough that is movable within the cam slot on the shaft. The movable jaw member has a second cam slot operably disposed thereon and operably coupled to the stationary cam pin on the shaft.

The present disclosure provides also provides an endoscopic forceps having a housing having a shaft that extends therefrom having a longitudinal axis defined therethrough. The shaft includes a stationary cam pin at a distal end thereof and an elongated cam slot operably disposed adjacent and in proximal relation relative to the stationary cam pin. An end effector assembly operatively connects to a distal end of the shaft and includes a pair of first and second jaw members that are pivotably coupled to one another. One of the first and second jaw members is movable relative to the other jaw member from an open position, wherein the first and second jaw members are disposed in spaced relation relative to one another, to a clamping position, wherein the first and second jaw members cooperate to grasp tissue therebetween. The movable jaw member has a drive pin operably coupled thereto and movable within the cam slot on the shaft from a proximal position that corresponds to the movable jaw member being in the clamping position, to a distal position that corresponds to the movable jaw member being in the open position. The movable jaw member has a second cam slot with an arcuate configuration defined thereon and operably coupled to the stationary cam pin on the shaft. A heat shrink extends substantially along a length of the shaft encasing the shaft including the cam slot thereon and drive pin therein to a position that is adjacent the stationary cam pin on the shaft.

The at least one movable jaw member may be both translatable along the longitudinal axis of the shaft and rotatable about the stationary cam pin upon actuation of the drive pin. The drive pin may be actuatable by a drive structure disposed through the shaft. The drive assembly may be operably coupled to the housing and includes a drive structure that operably couples to the drive pin, the drive structure configured such that proximal movement of a movable handle associated with the housing moves the drive structure proximally which causes the drive pin to actuate the movable jaw member to move from the open position to the clamping position and wherein distal movement of a movable handle moves the drive structure distally which causes the drive pin to actuate the movable jaw member to move from the clamping position back the open position.

As a generally applicable feature, the second cam slot may include a proximal end that extends below the elongated cam slot of the shaft and a distal end that extends above the elongated cam slot with respect to a central longitudinal axis of the shaft.

The stationary cam pin, the elongated cam slot, the drive pin and the second cam slot are arranged so that translation of the drive pin within the elongated cam slot along the longitudinal axis causes associated translation of the at least one movable jaw member as well as rotation of the at least one jaw member between the open and closed positions by way of a curved path of the elongated cam slot moving with respect to the stationary cam pin, the movement of the cam slot a result of the translation of the at least one jaw member.

### BRIEF DESCRIPTION OF THE DRAWING

Various embodiments of the present disclosure are described hereinbelow with references to the drawings, wherein:
FIG. 1A is a side, perspective view of an endoscopic bipolar forceps showing an end effector assembly including jaw members in an open configuration according to an embodiment of the present disclosure;
FIG. IB is a side, perspective view of an endoscopic bipolar forceps depicted in FIG. 1A with the jaw members in a closed position; and
FIGS. 2A-2C are schematic views of the jaw members depicted in FIGS. 1 and IB.

### DETAILED DESCRIPTION

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In the drawings and in the descriptions that follow, the term "proximal," as is traditional, will refer to an end that is closer to the user, while the term "distal" will refer to an end that is farther from the user.

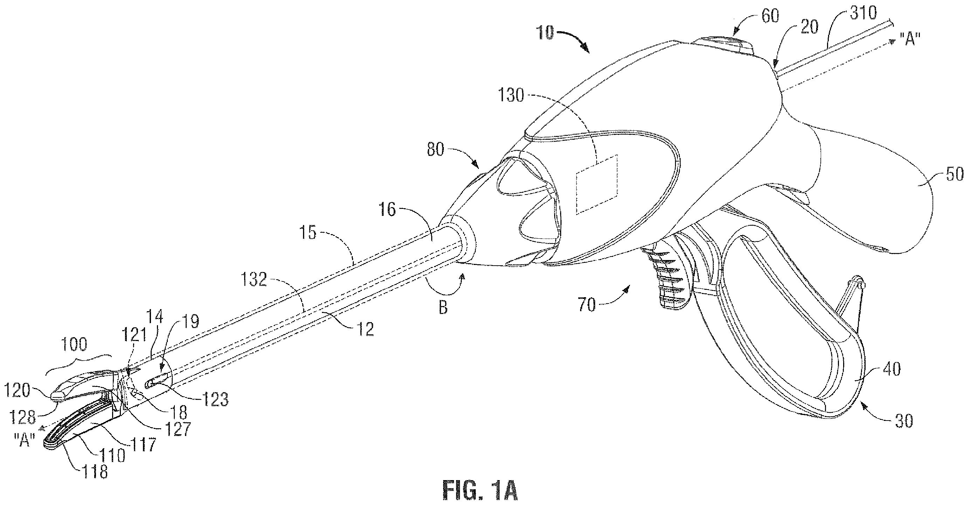

With reference to FIG. 1A, an illustrative embodiment of an electrosurgical apparatus, e.g., a bipolar forceps 10 (forceps 10) is shown. Forceps 10 is operatively and selectively coupled to an electrosurgical generator (not shown) for performing an electrosurgical procedure. As noted above, an electrosurgical procedure may include sealing, cutting, cauterizing, coagulating, desiccating, and fulgurating tissue all of which may employ RF energy. The electrosurgical generator may be configured for monopolar and/or bipolar modes of operation and may include or be in operative communication with a system that may include one or more processors in operative communication with one or more control modules (not shown) that are executable on the processor. The control module may be configured to instruct one or more modules to transmit electrosurgical energy, which may be in the form of a wave or signal/pulse, via one or more cables (e.g., an electrosurgical cable 310) to the forceps 10.

In certain embodiments, the forceps may be battery powered. In this instance, the forceps 10 is configured to function without the electrosurgical generator. Moreover, the control module may be disposed on or configured to operably couple to the forceps 10.

Forceps 10 is shown configured for use with various electrosurgical procedures and generally includes a housing 20, electrosurgical cable 310 that connects the forceps 10 to the electrosurgical generator, a rotating assembly 80 and a trigger assembly 70. For a more detailed description of the rotating assembly 80, trigger assembly 70, and electrosurgical cable 310 (including line-feed configurations and/or connections), reference is made to commonly-owned U.S. Patent Application Serial No. 11/595,194 filed on November 9, 2006, now U.S. Patent Publication No. 2007/0173814.

With continued reference to FIG. 1A, forceps 10 includes a shaft 12 that has a distal end 14 that is configured to mechanically engage an end effector assembly 100 operably associated with the forceps 10 and a proximal end 16 that mechanically engages the housing 20. A longitudinal axis "A-A" is defined through the shaft 12 (see FIG. 1A)

A stationary cam pin 18 of suitable configuration is operably disposed at a distal end 16 of the shaft 12 (FIGS. 1A-2B). Stationary cam pin 18 couples to a cam slot 121 that is operably disposed on one or both of a pair of jaw members 110 and 120 of the end effector 100, described in greater detail below.

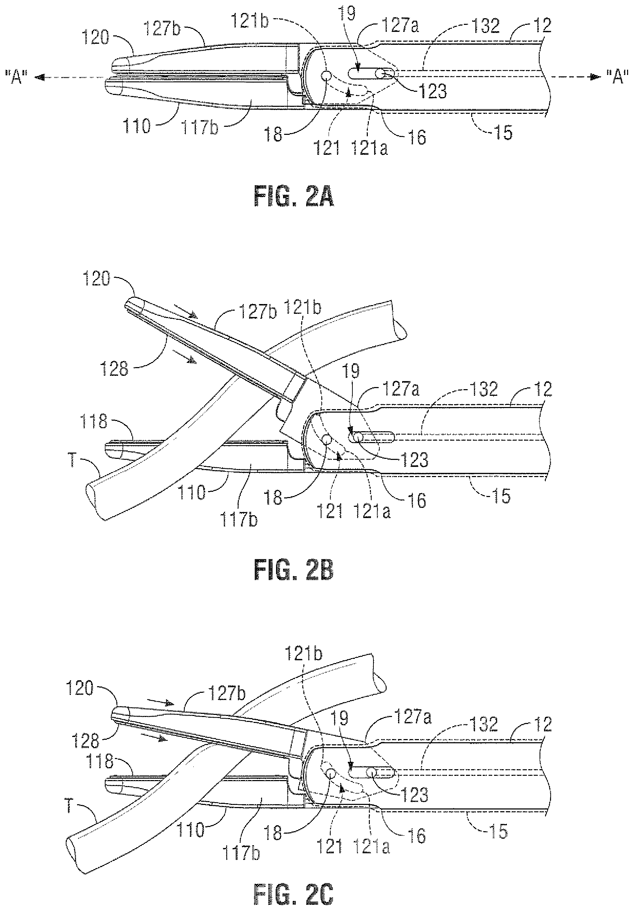

An elongated cam slot 19 (cam slot 19) of suitable configuration is operably disposed adjacent the stationary cam pin 18 (FIGS. 2A-2C). More particularly, cam slot 19 is configured to house a drive pin 123 that is operably coupled to the jaw member 120, described in greater detail below. Cam slot 19 is disposed in proximal relation relative to the stationary cam pin 18 (FIGS. 2A-2C). Cam slot 19 extends in parallel relation with respect to the longitudinal axis "A-A," as best seen in FIG. 2A. Having the cam slot 19 disposed in parallel relation with respect to the longitudinal axis "A-A" and in proximal relation relative to the stationary cam pin 18 facilitates translatable movement of the movable jaw member, e.g., jaw member 120, along the longitudinal axis "A-A" and rotatable movement of the jaw member 120 about the stationary cam pin 18 when the jaw member 120 is moved from the open position to the clamping position (and vice versa).

In the illustrated embodiment, the shaft 12 is encased with a heat shrink wrap 15 (heat shrink 15 shown phantomly illustrated). Specifically, heat shrink 15 extends substantially along a length of the shaft 12 encasing the shaft 12 (FIGS. 1A-1B). More specifically, the heat shrink 15 extends along the shaft 12 encasing the cam slot 19 including the drive pin 123 therein to a position that is adjacent the stationary cam pin 18 on the shaft 12 (as best seen in FIGS. 2A-2C), the significance of which described in greater detail below.

In certain embodiments, it may prove advantageous to coat the interior walls that define the cam slot 19 with one or more lubricious materials, e.g., polytetrafluoroethylene (PTFE), to facilitate movement of the drive pin 123 within the cam slot 19.

With reference again to FIG. 1A, handle assembly 30 includes a fixed handle 50 and movable handle 40. In one particular embodiment, fixed handle 50 is integrally associated with housing 20. Movable handle 40 is movable relative to fixed handle 50 for effecting movement of one or more components, e.g., driving structure 132, operably associated with drive mechanism 130 (FIG. 2A). Handle assembly 30 (including movable handle 40) may be configured such that proximal movement of the movable handle 40 "pulls" the driving structure 132, which, in turn, imparts movement of one or both of a pair of jaw members 110 and 120 from the normally open or neutral position (FIGS. 1A and 2B) to a clamping position (FIG. IB and 2A). Alternatively, handle assembly 30 (including movable handle 40 and drive mechanism 130) may be configured such that proximal movement of the movable handle 40 "pushes" the driving structure 132, which, in turn, imparts movement of the jaw members 110 and 120.

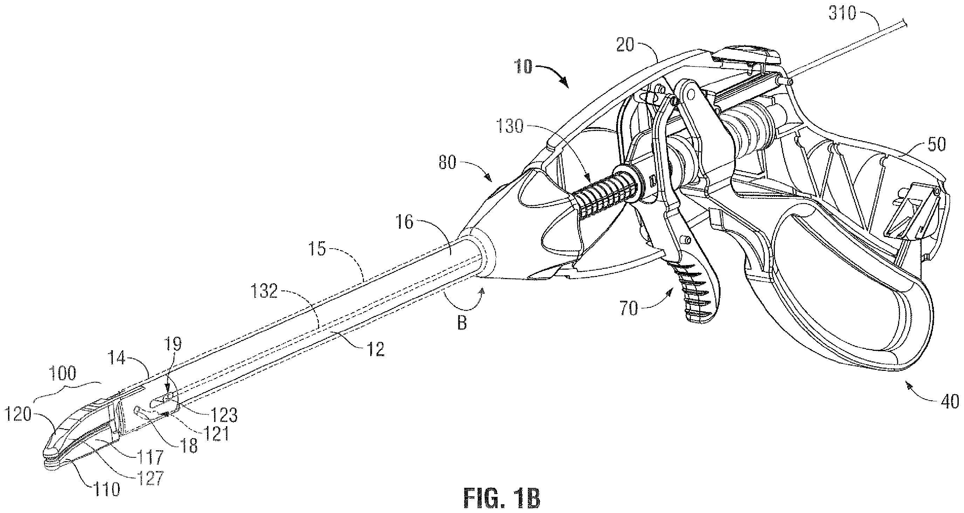

Drive mechanism 130 is in operative communication with movable handle 40 (see FIGS. 1A and 1B) for imparting movement of one or, in some instances, both of the jaw members 110, 120 of end effector assembly 100. More particularly, one or more suitable mechanical interfaces, e.g., a linkage interface, gear interface, or combination thereof operably couples the movable handle 40 to the drive mechanism 130. In the embodiment illustrated in FIGS. 1A-2C, proximal movement of the movable handle 40 moves the jaw member 120 toward each other from the normally opened position to the clamping position.

The driving structure 132 is configured such that proximal movement thereof causes the jaw member 120 to move from the open position (FIGS. 1A and 2B) to the clamping position (see FIG. 1B in combination with FIGS. 2A and 2C) and vice versa. To this end, driving structure 132 may be any suitable driving structure including but not limited to a wire, rod, cable, resilient band, etc. In the illustrated embodiment, driving structure 132 is in the form of a resilient drive rod of suitable proportion. Hereinafter, the driving structure is simply referred to as drive rod 132.

Drive rod 132 includes a proximal end (not explicitly shown) that is in operative communication with the movable handle 40 and a distal end that is operably coupled to the drive pin 123 of the jaw member 120 (FIGS. 2A-2C). The distal end of the drive rod 132 may operably couple to the drive pin 123 by any suitable methods, such as, for example, soldering, brazing and welding.

End effector assembly 100 is illustrated operably disposed at the distal end 14 of the shaft 12 (FIGS. 1A-2C). End effector assembly 100 includes opposing jaw members 110 and 120 that mutually cooperate to grasp, seal and, in some cases, divide large tubular vessels and large vascular tissues.

Jaw members 110 and 120 may be of the unilateral type, i.e., wherein one of the jaw members is moveable with respect to the other jaw member, or of the bilateral type, i.e., wherein both of the jaw members are movable with respect to each other. For illustrative purposes, the jaw members 110 and 120 are described in terms of the unilateral type.

Jaw members 110 and 120 include respective jaw housing 117 and 127 (FIG. 1A). Respective electrically conductive seal plates 118 and 128 are operably supported on and secured to respective distal ends 117b and 127b of jaw housings 117 and 127 (FIG. 2B). In one particular embodiment, the conductive seal plates 118 and 128 are secured to the respective distal ends 117b and 127b via an injection molding process. Jaw members 110 and 120 including respective jaw housings 117 and 127, and operative components associated therewith, may be formed from any suitable material, including but not limited to metal, metal alloys, plastic, plastic composites, and so forth. In one particular embodiment, the jaw members 110 and 120 are monolithically formed.

Jaw housing 127 and 117 of the respective jaw members 120 and 110 are substantially identical to each other. In view thereof, the operative features of jaw housing 127 are described in detail, and only those features that are unique to jaw housing 117 are described hereinafter.

With reference to FIGS. 2A and 2B, an embodiment of jaw housing 127 is illustrated. Jaw housing 127 includes the distal end 127b that is configured to operably support seal plate 128 and a proximal end 127a that operably couples to the distal end 14 of shaft 12.

Proximal end 127a includes a generally angled configuration (FIGS. 2A-2C) and is configured to move, e.g., pivot, from the open position (FIGS. 1A and 2B) to the closed or clamping position (FIGS. 1B, 2A and 2C). The angled configuration of the proximal end 127a facilitates moving the jaw member 120 from the open position to the clamping position.

The arcuate cam slot 121 (cam slot 121) is operably disposed at the proximal end 127a of the jaw member 120. The cam slot 121 couples to the stationary cam pin 18 and is configured such that when the jaw member 120 moves from the open position (FIGS. 1A and 2B) to the clamping position (FIGS. IB 2A and 2C), the jaw member 120 simultaneously translates proximally along the longitudinal axis "A-A" of the shaft 12 and rotates about the stationary cam pin 18. The simultaneous proximal translation and rotation of the jaw member 120 facilitates grasping tissue disposed between the jaw members 110 and 120 (FIG. 2C). Likewise, when the jaw member 120 moves from the clamping position to the open position, the jaw member 120 simultaneously translates distally along the longitudinal axis "A-A" of the shaft 12 and rotates about the stationary cam pin 18. The simultaneous distal translation and rotation of the jaw member 120 facilitates spreading tissue disposed adjacent the jaw members 110 and 120.

In the illustrated embodiment, cam slot 121 includes a proximal end 121a that extends below the cam slot 19 of the shaft 12 and a distal end 121b that extends above the cam slot 19 (see FIGS. 2A-2C). Positioning the cam slot 121 above and below the cam slot 19 maximizes translation and rotation of the jaw member 120 while minimizing exposure of the proximal end 127a through the heat shrink 15, as best seen in FIG. 2B. Minimizing exposure of the proximal end 127a through the heat shrink 15 minimizes and/or eliminates the likelihood of exposing the wiring coupled to the jaw member 120 and/or jaw member 110 to the surgical environment. As can be appreciated, minimizing and/or eliminating exposure of the wiring to the surgical environment is beneficial, especially in the instance of repeated cannula insertion/retraction cycles. That is, there is less likelihood of the wiring contacting or scrapping against the cannula and/or operative component associated therewith.

The drive pin 123 is operably disposed at predetermined position on the proximal end 127a of the jaw member 120 and is housed within the cam slot 19 of the shaft 12. The drive pin 123 is configured to translate within the cam slot 19 of the shaft 12 when the drive rod 132 is moved proximally and distally in response to respective proximal and distal movement of the movable handle 40.

In an assembled configuration, cam pin 18 is positioned within an opening of the jaw member 110 and the cam slot 121 of the jaw member 120. Once assembled, the jaw members 120 and/or jaw member 110 may be pivotably supported at the distal end 14 of the shaft 12 by any suitable method, such as, for example, by the method described in commonly-owned U.S. Pat. No. 7,597,693 to Garrison.

In use, jaw members 110 and 120 are, initially, in the open position (see FIGS. 1A and 2B). Tissue is positioned between the jaw member 110 and 120. Movable handle 40 is moved proximally (FIG. 1B), which, in turn, causes the drive rod 132 to move proximally. Proximal movement of the drive rod 132 moves the drive pin 123 proximally within the cam slot 19 causing the jaw member 120 to pivot about the stationary cam pin 18. As the jaw member 120 rotates about the cam pin 18, the jaw member 120 translates proximally (illustrated by directional arrows in FIGS. 2B and 2C) a predetermined distance to the clamping position (FIG. 2A). As noted above, this proximal movement of the jaw member 120 facilitates grasping and squeezing tissue between the jaw members 110 and 120. In the clamping position, and in one particular embodiment, a closure force in the range from about 3kg/cm² to about 16 kg/cm² is present at the jaw members 110 and 120. Thereafter, tissue is electrosurgically treated, e.g., tissue is sealed. A closure force in the range of 3kg/cm² to about 16 kg/cm² may provide a uniform and consistent seal across the tissue. Subsequently, movable handle 40 is released and the jaw member 120 rotates about the cam pin 18 and translates distally a predetermined distance back to the open position (FIG. 2B).

As noted above, the jaw member 120 may be utilized to separate and/or spread tissue. Accordingly, and in one particular surgical scenario, prior to positioning tissue between the jaw members 110 and 120 and/or in the instance where more than one tissue specimen needs to be electrosurgically treated, a user may repeatedly open and close the jaw member 120 to spread tissue. That is, the distal translation of the jaw member 120 may be utilized to separate and/or spread tissue apart. As can be appreciated, this may prove advantageous such as, for example, where the surgeon is required to electrosurgically treat tissue that is at a compromised angle or position.

The unique configuration of the jaw member 120 including a movable drive pin 123 that is operably coupled to the cam slot 19 disposed on the shaft 12, in addition to the jaw member 120 including an arcuate cam slot 121 that houses the stationary cam pin 18 diminishes and/or eliminates the occurrence of the proximal end 127a, i.e., "flag", extending past the profile of shaft 12. As a result thereof, the likelihood of the wiring that is coupled to the jaw member 120 being exposed during the surgical procedure is greatly reduced and/or eliminated. Moreover, the frictional losses that are typically transferred to the drive rod 132 when the shaft is bent or articulated and when the "flags" extend past the shaft 12 is greatly reduced and/or eliminated.

In accordance with the present disclosure, a method of manufacture for an electrosurgical instrument, e.g., forceps 10, is disclosed. Elongated slot 19 for cam or drive pin 123 is formed at the distal end 4 of shaft 12. Arcuate slot 121 for cam pin 18 is formed on the jaw member 120. The elongated slot 19 and drive pin 123 are coupled to the cam pin 18 and arcuate slot 121, respectively, such that the jaw member 120 is both translatable proximally along the longitudinal axis "A-A" of the shaft 12 and rotatable about the stationary drive pin 18 to facilitate grasping and squeezing tissue when the jaw member 120 moves from the open position to the clamping position. Moreover, the jaw member 120 is translatable distally along the longitudinal axis "A-A" of the shaft 12 and rotatable about the stationary drive pin 18 to facilitate spreading tissue when the jaw member 120 moves from the clamping position back to the open position.

From the foregoing and with reference to the various figure drawings, those skilled in the art will appreciate that certain modifications can also be made to the present disclosure without departing from the scope of the same. For example, it is contemplated that in certain instances one or more resilient members, e.g., compression spring (not shown), may be operably associated with or coupled to one or both of the jaw members 110 and 120. In this instance, the spring may be configured to provide a specific clamping force or seal force between the jaw members 110 and 120 when the jaw members 110 and 120 are in the clamping position.

It is contemplated that in certain instances, one or both seal surface of the seal plates 128 and 118 may be textured or otherwise treated to facilitate grasping or spreading tissue.

The invention is set out in the appended claims.

## Claims

1. An endoscopic forceps (10), comprising:
a housing (20) having a shaft (12) that extends therefrom having a longitudinal axis defined therethrough, the shaft including a stationary cam pin (18) at a distal end thereof and an elongated cam slot (19) operably disposed adjacent and in proximal relation relative to the stationary cam pin; and
an end effector assembly (100) operatively connected to a distal end of the shaft and including a pair of first and second jaw members (110, 120) pivotably coupled to one another, the second of the jaw members (120) translatable along the longitudinal axis and rotatable about the stationary cam pin, from an open position, wherein the first and second jaw members are disposed in spaced relation relative to one another, to a clamping position, wherein the first and second jaw members cooperate to grasp tissue therebetween; and
the at least second jaw member having a drive pin (122) operably coupled thereto and movable within the cam slot on the shaft, the second of the jaw members having a second cam slot (121) operably disposed thereon and operably coupled to the stationary cam pin on the shaft, the stationary cam pin of the shaft positioned within an opening of the first of the jaw members (110) and within the second cam slot of the second jaw member (120).

2. An endoscopic forceps according to claim 1, wherein the drive pin is movable within the cam slot on the shaft from a proximal position that corresponds to the second jaw member being in the clamping position, to a distal position that corresponds to the second jaw member being in the open position.

3. An endoscopic forceps according to claim 1 or 2, wherein the second cam slot on the second jaw member includes an arcuate configuration.

4. An endoscopic forceps according to claim 1, 2 or 3, wherein the second jaw member is both translatable along the longitudinal axis of the shaft and rotatable about the stationary cam pin upon actuation of the drive pin.

5. An endoscopic forceps according to claim 1, 2, 3 or 4, wherein a heat shrink (15) extends substantially along a length of the shaft encasing the shaft including the cam slot thereon and drive pin therein to a position that is adjacent the stationary cam pin on the shaft.

6. An endoscopic forceps according to claim 1, 2, 3, 4 or 5, wherein a drive assembly is operably coupled to the housing and includes a drive structure (132) that operably couples to the drive pin, the drive structure configured such that proximal movement of a movable handle (140) associated with the housing moves the drive structure proximally which causes the drive pin to actuate the second jaw member to move from the open position to the clamping position and wherein distal movement of a movable handle moves the drive structure distally which causes the drive pin to actuate the second jaw member to move from the clamping position back the open position.

## Patentansprüche

1. Endoskopische Zange (10), die umfasst:
ein Gehäuse (20) mit einem Schaft (12), der sich von dort aus erstreckt und längs durch diesen hindurch eine Längsachse definiert, wobei der Schaft an seinem distalen Ende einen stationären Nockenstift (18) und einen langgestreckten Nockenspalt (19), der benachbart und in proximaler Beziehung relativ zu dem stationären Nockenstift betreibbar angeordnet ist, enthält; und
eine Greiforgananordnung (100), die betreibbar mit einem distalen Ende des Schafts verbunden ist und die ein Paar Klemmbackenelemente, bestehend aus einem ersten und einem zweiten Klemmbackenelement (110, 120) enthält, die miteinander drehbar gekoppelt sind, wobei das zweite der Klemmbackenelemente (120) eine Translationsbewegung entlang der Längsachse und eine drehbare Bewegung um den stationären Nockenstift von einer geöffneten Position, in der das erste und das zweite Klemmbackenelement in einem beabstandeten Verhältnis relativ zueinander angeordnet sind, in eine Klemmposition, in der das erste und das zweite Klemmbackenelement zusammenwirken, um Gewebe zwischen ihnen zu greifen, ausführen kann; und
wobei das mindestens zweite Klemmbackenelement einen Antriebsstift (122) aufweist, der mit demselben betreibbar gekoppelt ist und innerhalb des Nockenspalts auf dem Schaft beweglich ist, wobei das zweite der Klemmbackenelemente einen zweiten Nockenspalt (121) aufweist, der auf demselben betreibbar angeordnet ist und mit dem stationären Nockenstift auf dem Schaft betreibbar gekoppelt ist, wobei der stationäre Nockenstift des Schafts innerhalb einer Öffnung des ersten der Klemmbackenelemente (110) und innerhalb des zweiten Nockenspalts des zweiten Klemmbackenelements (120) positioniert ist.

2. Endoskopische Zange nach Anspruch 1, wobei der Antriebsstift innerhalb des Nockenspalts auf dem Schaft von einer proximalen Position, die dem zweiten Klemmbackenelement, das sich in der Klemmposition befindet, entspricht, in eine distale Position, die dem zweiten Klemmbackenelement, das sich in der geöffneten Position befindet, entspricht, beweglich ist.

3. Endoskopische Zange nach Anspruch 1 oder 2, wobei der zweite Nockenspalt auf dem zweiten Klemmbackenelement eine bogenförmige Konfiguration enthält.

4. Endoskopische Zange nach Anspruch 1, 2 oder 3, wobei das zweite Klemmbackenelement sowohl eine Translationsbewegung entlang der Längsachse des Schafts als auch eine drehbare Bewegung um den stationären Nockenstift bei Betätigung des Antriebsstifts ausführen kann.

5. Endoskopische Zange nach Anspruch 1, 2, 3 oder 4, wobei sich eine Wärmeschrumpfung (15), die den Schaft, der den Nockenspalt darauf und den Antriebsstift in demselben enthält, ummantelt, im Wesentlichen entlang einer Länge des Schafts bis zu einer Position, die zu dem stationären Nockenstift auf dem Schaft benachbart ist, erstreckt.

6. Endoskopische Zange nach Anspruch 1, 2, 3, 4 oder 5, wobei eine Antriebsanordnung mit dem Gehäuse betreibbar gekoppelt ist und eine Antriebsstruktur (132) enthält, die betreibbar an den Antriebsstift ankoppelt, wobei die Antriebsstruktur derart konfiguriert ist, dass eine proximale Bewegung eines beweglichen Griffs (140), der mit dem Gehäuse verbunden ist, die Antriebsstruktur proximal bewegt, was den Antriebsstift veranlasst, das zweite Klemmbackenelement zu betätigen, damit sich dieses von der geöffneten Position in die Klemmposition bewegt, und wobei eine distale Bewegung eines beweglichen Griffs die Antriebsstruktur distal bewegt, was den Antriebsstift veranlasst, das zweite Klemmbackenelement zu betätigen, damit sich dieses von der Klemmposition zurück in die geöffnete Position bewegt.

## Revendications

1. Forceps endoscopique (10), comprenant :
un logement (20) ayant un arbre (12) qui s'étend de celui-ci et ayant un axe longitudinal qui y est défini, l'arbre comprenant une broche de came stationnaire (18) à son extrémité distale et une fente de came allongée (19) disposée en service adjacente à la broche de came stationnaire et en relation proximale avec celle-ci ; et
un ensemble effecteur terminal (100) raccordé en service à une extrémité distale de l'arbre et comprenant une paire de premier et second éléments de mors (110, 120) couplés à pivotement l'un à l'autre, le second des éléments de mors (120) pouvant se déplacer en translation le long de l'axe longitudinal et tourner autour de la broche de came stationnaire d'une position ouverte, dans laquelle les premier et second éléments de mors sont disposés en relation espacée l'un de l'autre, à une position de serrage, dans laquelle les premier et second éléments de mors coopèrent pour saisir un tissu entre eux ; et
le au moins un second élément de mors ayant une broche d'entraînement (122) qui lui est couplée en service et peut se déplacer dans la fente de came sur l'arbre, le second des éléments de mors ayant une seconde fente de came (121) qui y est disposée en service et est couplée en service à la broche de came stationnaire sur l'arbre, la broche de came stationnaire de l'arbre étant positionnée dans une ouverture du premier des éléments de mors (110) et dans la seconde fente de came du second élément de mors (120).

2. Forceps endoscopique selon la revendication 1, dans lequel la broche d'entraînement est mobile dans la fente de came sur l'arbre d'une position proximale, qui correspond au second élément de mors qui se trouve en position de serrage, à une position distale, qui correspond au second élément de mors qui se trouve en position ouverte.

3. Forceps endoscopique selon la revendication 1 ou 2, dans lequel la seconde fente de came sur le second élément de mors présente une configuration arquée.

4. Forceps endoscopique selon la revendication 1, 2 ou 3, dans lequel le second élément de mors peut à la fois effectuer une translation le long de l'axe longitudinal de l'arbre et tourner autour de la broche de came stationnaire lors de l'actionnement de la broche d'entraînement.

5. Forceps endoscopique selon la revendication 1, 2, 3 ou 4, dans lequel une gaine thermorétractable (15) s'étend sensiblement sur la longueur de l'arbre en enserrant l'arbre, notamment la fente de came qui y est ménagée et la broche d'entraînement qui s'y trouve dans une position qui est adjacente à la broche de came stationnaire sur l'arbre.

6. Forceps endoscopique selon la revendication 1, 2, 3, 4 ou 5, dans lequel un ensemble d'entraînement est couplé en service au logement et comprend une structure d'entraînement (132) qui se couple en service à la broche d'entraînement, la structure d'entraînement étant configurée de sorte que le mouvement proximal d'une poignée mobile (140) associée au logement déplace la structure d'entraînement de manière proximale, ce qui amène la broche d'entraînement à actionner le second élément de mors pour le déplacer de la position ouverte à la position de serrage et dans lequel le mouvement distal d'une poignée mobile déplace la structure d'entraînement de manière distale, ce qui amène la broche d'entraînement à actionner le second élément de mors pour le redéplacer de la position de serrage à la position ouverte.
